# EUROPÄISCHE PATENTANMELDUNG

(11) **EP 1 709 929 A1**
(43) Veröffentlichungstag der Anmeldung: **11.10.2006**
(21) Anmeldenummer: 06007376.4
(22) Anmeldetag: 07.04.2006
(51) Int. Cl.: A61C 1/08, A61C 13/12, B23Q 1/54

(54) **Arbeitstisch für Zahntechniker**

(30) Priorität: 08.04.2005 DE 102005016396
(71) Anmelder: Dr.-medic-stom./UMF Temeschburg, Herbert Hatzlhoffer, 76337 Waldbronn (DE)
(72) Erfinder: Dr.-medic-stom./UMF Temeschburg, Herbert Hatzlhoffer, 76337 Waldbronn (DE)

(57) **Zusammenfassung**

Die vorliegende Erfindung betrifft einen Arbeitstisch für Zahntechniker und Zahnärzte und insbesondere zur Erstellung einer Implantatsbohrschablone, wobei eine Werkzeugaufnahme (13) relativ zu einer unteren Grundplatte (2) um zumindest zwei Schwenkachsen (A, B) und entlang von zumindest zwei Translationsachsen (Y, W) verstellbar angeordnet ist, wobei die beiden Schwenkachsen in einer gemeinsamen horizontalen Ebene liegen und sich unter einem Winkel von 90° schneiden, und wobei die erste Translationsachse (Y) in Horizontalrichtung orientiert ist und wobei die zweite Translationsachse (Z) parallel zu oder identisch mit einer Werkzeugachse (W) ist. Hierbei schneiden sich erfindungsgemäß die beiden Schwenkachsen (A, B) und die Werkzeugachse (W) in einem gemeinsamen Schnittpunkt (O).

## Beschreibung

Die Erfindung betrifft einen Arbeitstisch für Zahntechniker und Zahnärzte und insbesondere zur Erstellung einer Implantats-Bohrschablone mit einer ein Werkstück zumindest mittelbar tragenden Grundplatte und einer relativ zur Grundplatte beweglich angeordneten Werkzeugaufnahme für ein Werkzeug, wie insbesondere einen Bohrer, mit einer Werkzeugachse W, wobei die Werkzeugaufnahme relativ zur Grundplatte um zumindest zwei horizontale Schwenkachsen A, B und entlang von zumindest zwei Translationsachsen Y, Z verstell- und bewegbar angeordnet ist, wobei die beiden Schwenkachsen A, B in einer gemeinsamen Horizontalebene liegen und sich unter einem Winkel in der Größenordnung von 90° schneiden und wobei die erste Translationsachse Y in Horizontalrichtung orientiert ist und die zweite Translationsachse Z entlang der Werkzeugachse W verläuft.

Ein entsprechender Arbeitstisch ist beispielsweise aus der EP-A-1 520 551 bekannt, bei welchem die Werkzeugaufnahme gegenüber dem Werkstück um zumindest vier Schwenkachsen schwenkbar und in Richtung zumindest einer Translationsachse verschieblich angeordnet ist, um so die Werkzeugaufnahme einerseits relativ zum Werkstück und andererseits relativ zum Benutzer in beliebige Positionen bringen zu können. Mit Hilfe dieser bekannten Vorrichtung ist ein ergonomisch optimales Arbeiten möglich, wobei der Benutzer an einem festen Arbeitsplatz verbleiben kann, dem die unverstellbare untere Grundplatte zugeordnet ist und wobei er die Position der Werkzeugaufnahme bezogen auf sich selbst beliebig verändern kann.

Das Herstellen einer Implantats-Bohrschablone erfolgt bei der bekannten Vorrichtung entsprechend der folgenden Verfahrensschritte: Positionieren eines Kiefermodells eines Patienten an dem Arbeitstisch; Modellieren einer Röntgenschablone zumindest eines Teils des Kiefermodells unter Einfügung von zumindest drei Referenzpunkten (so genannten Landscapes) mit vorgegebenem gegenseitigen Abstand sowie vorgegebener Position in Bezug auf den Arbeitstisch; Erstellen von Röntgenbildern des mit einem implantat zu versehenden Kiefers des Patienten mit eingesetzter Röntgenschablone; Vermessen und Auswerten der Röntgenbilder durch eine elektronische Auswertevorrichtung, insbesondere durch einen Computer und Erzeugung von Messdatensätzen; Planen und Ermitteln des zu verwendenden Zahnimplantats und dessen Position (Ort, Winkel, Tiefe); Errechnen aus der Position des Zahnimplantats die Koordinaten des in die Bohrschablone einzubringenden Bohrlochs; Umrechnen der errechneten Koordinaten in die Winkelstellungen der vier Schwenkachsen und der Verschiebestellung der ersten Translationsachse des Arbeitstischs zur arbeitstischbezogenen Einstellung der Bohrposition der Werkzeugaufnahme; Modellieren einer Bohrschablone zumindest eines Teils des Kiefermodells oder Aufsetzen der als Bohrschablone zu verwendenden Röntgenschablone auf das Kiefermodell; Einstellen der Winkelstellungen der vier Schwenkachsen und der Verschiebestellung der ersten Translationsachse, um die Werkzeugaufnahme gegenüber der Bohrschablone in die errechnete Stellung zu bringen; und Bohren des Bohrlochs in die Bohrschablone unter Verwendung eines in die Werkzeugaufnahme eingesetzten Bohrwerkzeugs.

Von diesem bekannten Arbeitstisch ausgehend liegt der vorliegenden Erfindung die Aufgabe zugrunde, einen Arbeitstisch der eingangs genannten Art hinsichtlich seiner Genauigkeit bezüglich der Arbeits- bzw. Verstellmöglichkeiten zu verbessern.

Diese Aufgabe wird erfindungsgemäß dadurch gelöst, dass sich die beiden Schwenkachsen A, B und die Werkzeugachse W in einem gemeinsamen Schnittpunkt O schneiden. Hierdurch ergibt sich der Vorteil, dass ein Verschwenken des Werkzeugs um eine der beiden Schwenkachsen A, B in erster Linie nur zu einem Ändern des Schwenkwinkels α, β, also der Orientierung des Werkzeugs führt, ohne jedoch das Werkzeug und insbesondere den Bearbeitungsbereich des Werkzeugs, also beispielsweise die Bohrerspitze, um Streckenlängen x, y zu weit aus der Nullstellung herauszuschwenken. Denn bei exzentrischen Anordnungen der Werkzeugachse W gegenüber einer oder beiden Schwenkachsen A, B führt ein Verschwenken zu einer erheblichen Positionsänderung des Bearbeitungsbereichs des Werkzeugs um die Streckenlängen x, y, was das Einstellen der Arbeitsposition und das Arbeiten mit einem solchen Werkzeug aufgrund hierbei erforderlicher Nachjustierungen (insbesondere bezüglich von Translationsachsen X, Y) unnötig erschwert.

Besonders vorteilhaft ist es, wenn die erste Translationsachse, also die Y-Achse, entlang der ersten Schwenkachse A und somit ebenfalls durch den genannten gemeinsamen Schnittpunkt verläuft, wobei diese Translationsachse Y diejenige Bewegungsrichtung definiert, der zumindest die Werkzeugaufnahme, insbesondere aber auch ein die Werkzeugaufnahme zumindest mittelbar tragender, horizontal verschiebbarer Auslegerarm (idealerweise mit seiner Mittelachse) gegenüber der Grundplatte folgt. Hierdurch lässt sich die räumliche Bewegung des Werkzeugs relativ zur Grundplatte in einfache kartesische Koordinaten zerlegen, denn bei einem Verschwenken um die zweite Schwenkachse B bewegt sich die vertikale Werkzeugachse entlang der Y-Richtung und kompensiert bzw. verstärkt hierdurch translatorische Verstellungen in Y-Richtung.

Darüber hinaus sollte auch dieser genannte Auslegerarm, zumindest aber die Werkzeugaufnahme gegenüber der Grundplatte in einer zur Y-Achse senkrechten horizontalen X-Richtung entlang einer dritten Translationsachse, also der X-Achse, verstellt werden können, um hierdurch alle erforderlichen Arbeitspositionen des Arbeitstisches bzw. des darauf positionierten Werkstücks mit Hilfe der Werkzeugaufnahme bzw. des Werkzeugs anfahren zu können.

Somit kann der Benutzer, vor allem wenn er von einem Rechner die Sollwerte für alle verstellbaren Koordinaten vorgegeben bekommt, schnell und auf einfache Weise das Werkzeug in Position bringen, um dann den Bearbeitungsvorgang durchführen zu können. Soweit beispielsweise aus der US-A 6 634 883 ein Arbeitstisch bekannt ist, bei dem zwar der Auslegerarm zwei horizontale Schwenkachsen trägt, die horizontalen Translationsachsen jedoch der Modellaufnahmeplatte zugeordnet sind und die Platte bzw. das auf ihr angeordnete Werkstück/Modell (ohne eigene Schwenkmöglichkeit) in X- bzw. Y-Richtung verstellt werden muss, so verkompliziert dies das Arbeiten mit dem Arbeitstisch. Denn das Verschwenken um die Schwenkachsen muss an der Werkzeugaufnahme erfolgen, während das horizontale Verstellen bzw. Korrigieren an der auf der Grundplatte aufgelagerten Modellaufnahmeplatte erfolgen muss. Folglich hat man bei diesem Stand der Technik keinen festen Bezugspunkt des Werkstücks, von dem ausgehend man die Werkzeugaufnahme einstellen kann. Vielmehr muss man alle verstellbaren Komponenten, die sich jeweils gegenseitig beeinflussen, quasi gleichzeitig solange einstellen, bis man die gewünschte Arbeitsposition erreicht hat. Zusätzlich bilden bei diesem Stand der Technik auch die beiden horizontalen Schwenkachsen keinen gemeinsamen Schnittpunkt mit der exzentrisch gegenüber einer der beiden Schwenkachsen angeordneten Werkzeugachse, was zu den eingangs geschilderten Nachteilen führt.

Schließlich ist erfindungsgemäß vorgesehen, dass die Grundplatte eine Modellaufnahme für das Werkstück aufweist, die wiederum um eine weitere, sich senkrecht zur Grundplatte erstreckende vertikale Schwenkachse C drehbar gelagert ist, um so das Werkstück auch relativ zur Werkzeugaufnahme bzw. zum Werkzeug um die C-Achse um bis zu 360° schwenken zu können. Diese durch das Werkstück laufende Schwenkachse C bildet bei dem Erfindungsgegenstand genau einen solchen festen Bezugspunkt, der dem Stand der Technik fehlt. Stellt man die X-Achse und die Y-Achse bzw. die A-Achse so ein, dass die A-Achse und die B-Achss durch die C-Achse verlaufen und die W- bzw. Z-Achse mit der C-Achse zusammenfällt, so hat man den "absoluten Nullpunkt" des Arbeitstischsystems, so dass sich alle Koordinaten der Werkzeugaufnahme, also Ist- und Sollwerte beim Bearbeiten eines Werkstücks auf diese Nulleinstellungen beziehen lassen und so auch reproduzierbar sind.

Die genannten Schwenk- und Translationsachsen sind zweckmäßigerweise mit Arretiermitteln kombiniert, um eine einmal eingestellte Schwenk- bzw. Translationsposition während des gesamten Arbeitsvorgangs beibehalten zu können. Lediglich die erste Translationsachse Z, die der Werkzeugachse W entspricht, muss für den Bearbeitungsvorgang, also insbesondere für das Bohren eines Bohrlochs in die Bohrschablone in Richtung der Werkzeugachse beweglich sein, zumindest soweit beweglich, bis ein vorgegebener Anschlag erreicht ist.

Die Arretiermittel bestehen zweckmäßigerweise aus Kegelklemmungen, um über großflächige konische Flächenpressungen und nicht nur über Punktpressungen eine sichere und unverfälschte Festlegung zu ermöglichen.

Um die Position der verschiedenen Achsen besser einstellen und überwachen zu können, empfiehlt es sich des Weiteren, die Achsen zu digitalisieren, also hieran Bewegungsaufnehmer anzuordnen. Hierdurch können die einstellbaren und einzustellenden Koordinaten, die beispielsweise von einer Diagnosesoftware als Sollwert für die Position und Orientierung des jeweiligen Bohrlochs in der Bohrschablone vorgegeben werden können, durch entsprechende Anzeigemittel, die den jeweiligen istwert anzeigen, beim Verstellen der verschiedenen Achsen berücksichtigt werden, bis schließlich alle lst-Werte den vorgegebenen Sollwert entsprechen. In diesem Zusammenhang empfiehlt es sich, wenn die von der Diagnosesoftware ermittelten Daten im ASCII-Format vorliegen, in eine Sollwerttabelle übernommen werden können und so den ebenfalls im ASCII-Format vorliegenden Istwerten gegenübergestellt werden können.

Das Verstellen der verschiedenen Achsen und somit das Ändern der Ist-Werte zum Erreichen der Sollwerte kann zum einen manuell erfolgen; ebenso ist es aber auch möglich, dass an den Achsen Stell- bzw. Servomotoren vorgesehen sind, die die Verstellbewegungen in Schwenk- oder Translationsrichtung durchführen. ln diesem Fall wäre ein automatisiertes Einstellen der Bohrposition bis hin zum sogar automatisierten Bohren möglich.

Sowohl beim manuellen als auch beim maschinellen Verstellen kann der Benutzer beim Erreichen der vorgegebenen Sollwerte ein (beispielsweise optisches oder akustisches) Freigabesignal erhalten, woraufhin er den Bearbeitungsvorgang beginnen und das Bohrloch erstellen kann. Hat während des Bearbeitungsvorgangs auch das Werkzeug entlang der Z-Achse seinen Sollwert erreicht, so kann der Benutzer durch eine weitere optische oder akustische Anzeige hierüber informiert werden und das Bearbeiten abbrechen.

Besonders vorteilhaft an der Digitalisierung der Daten ist die Tatsache, dass diese Istwerte in einer so genannten Blackbox abgespeichert werden können, um hierdurch - ohne Verwaltungsaufwand und Manipulationsmöglichkeiten - diese Istwerte auch noch Wochen bzw. Monate nach dem Bearbeitungsvorgang reproduzierbar abrufen zu können, wodurch der Benutzer die von ihm beim Bearbeiten verwendeten istwerte entsprechend belegen kann.

Weitere Merkmale und Vorteile der vorliegenden Erfindung ergeben sich aus der nachfolgenden Beschreibung eines Ausführungsbeispiels anhand der Zeichnungen; hierbei zeigen
- Figur 1: einen erfindungsgemäßen Arbeitstisch in Seitenansicht;
- Figur 2: den Arbeitstisch aus Figur 1 in Vorderansicht; und
- Figur 3: den Arbeitstisch aus den Figuren 1 und 2 in Draufsicht.

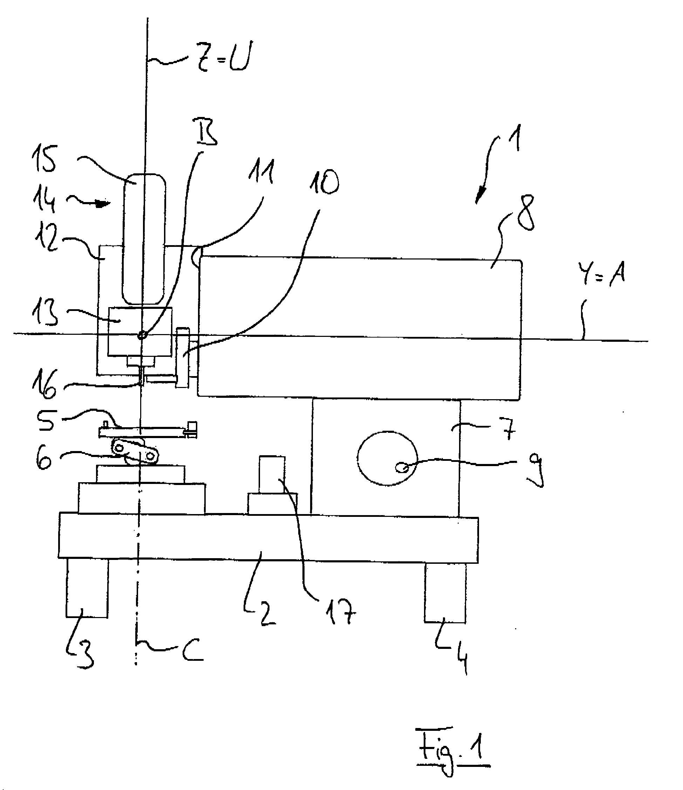

In den Figur 1, 2 und 3 ist ein Arbeitstisch 1 dargestellt, der eine horizontale untere Grundplatte 2 aufweist, die fest auf einem (nicht dargestellten) Untergrund wie etwa einem Labortisch etc. über Füße 3, 4 aufgestellt werden kann. Die Grundplatte trägt zum einen eine Modellaufnahmeplatte 5, auf der ein (nicht dargestelltes) Werkstück bzw. Modell festlegbar ist, wobei die Position der Modellaufnahmeplatte 5 gegenüber der Grundplatte 2 über Artikulatoren 6 einerseits und über die schwenkbare Lagerung um eine sich senkrecht zur Grundplatte 2 erstreckende vertikale Drehachse C andererseits nahezu beliebig verstellbar ist.

Des Weiteren trägt die Grundplatte 2 auf ihrer Oberseite einen etwa quaderförmigen Sockel 7, der auf seiner Oberseite einen quaderförmigen Auslegerarm 8 trägt, der gegenüber dem Sockel 7 in Horizontalrichtung entlang einer Achse X verfahrbar angeordnet ist. Das horizontale Verfahren erfolgt über einen Spindelantrieb, der durch das Bezugszeichen 9 angedeutet ist und der aus einer sich innerhalb des Sockels 7 erstreckenden Gewindespindel besteht, die mit einer entsprechenden Aufnahme des Auslegerarms 8 zusammenwirkt. Der Auslegerarm 8 wiederum ist gegenüber dem Sockel 7 in einer weiteren horizontalen Richtung Y zusätzlich verfahrbar, wobei die erste Translationsachse X im Wesentlichen senkrecht zur zweiten Translationsachse Y angeordnet ist. Auch diese Verfahrbewegung wird über einen schematisch dargestellten Spindelantrieb 10 durchgeführt, der ähnlich dem Spindelantrieb 9 des Sockels ausgeführt ist und bei dem wiederum eine (nicht dargestellte) Gewindespindel sich horizontal durch den Auslegerarm 8 erstreckt und mit dem Sockel 7 zusammenwirkt.

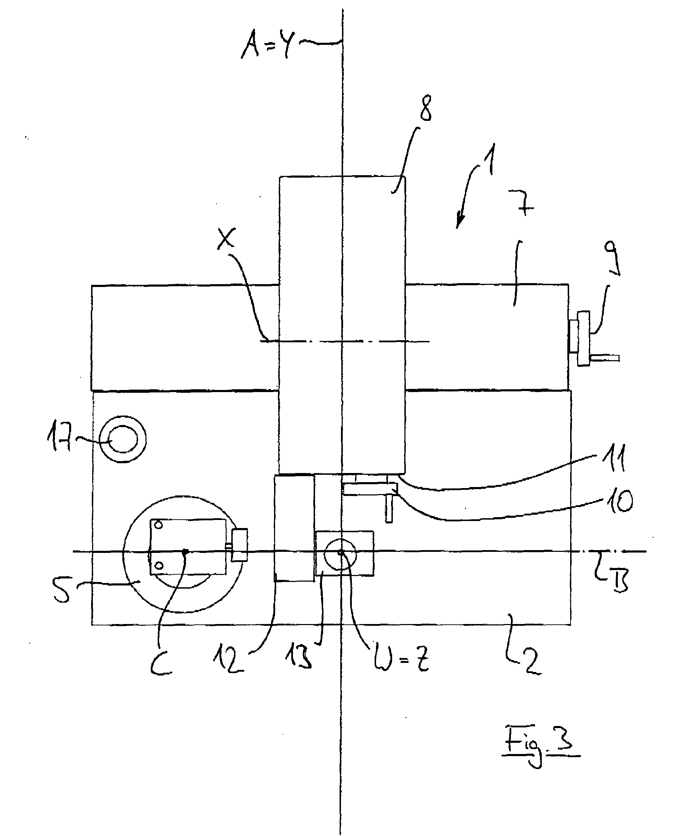

Wie insbesondere aus Figur 3 ersichtlich ist, trägt der Auslegerarm 8 an einer vorderen Stirnseite 11 einen außermittig festgelegten Exzenterarm 12, der wiederum seitlich eine Werkzeugaufnahme 13 mit einer parallel zum Exzenterarm 12 angeordneten Werkzeugachse W trägt. Die Werkzeugaufnahme ist zusammen mit dem Exzenterarm 12 gegenüber dem Auslegerarm 8 um eine horizontale Schwenkachse A schwenkbar gelagert, wobei die Schwenkachse A in Richtung der ersten Translationsachse Y verläuft. Der außermittig zur Schwenkachse A (und auch zur Mittelachse des Auslegerarms 8) angeordnete Exzenterarm 12 sorgt dafür, dass sich die Schwenkachse A und die Werkzeugachse W in einem gemeinsamen Schnittpunkt O schneiden.

Die Werkzeugaufnahme 13 ist des weiteren um eine horizontale Schwenkachse B gegenüber dem Exzenterarm 12 schwenkbar gelagert, wobei die Schwenkachse B senkrecht zur Translationsachse Y des Auslegerarms 8, senkrecht zur Schwenkachse A, senkrecht zur Werkzeugachse W und auch senkrecht zur Haupterstreckungsebene des Exzenterarms 12 angeordnet ist und ebenfalls durch den genannten Schnittpunkt O verläuft. Hierdurch lässt sich sicherstellen, dass ein Verschwenken der Werkzeugaufnahme zu einer minimalen seitlichen Auslenkung führt.

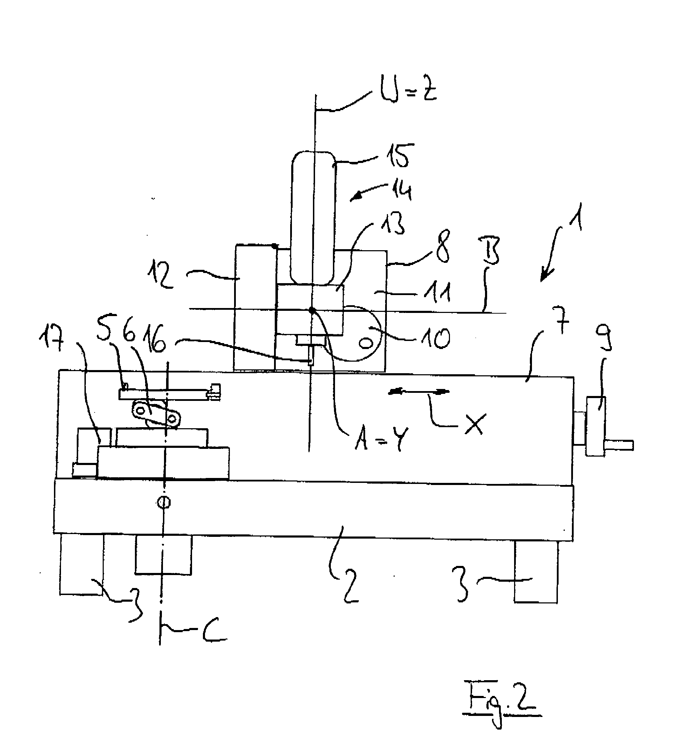

ln den Figuren 1 und 2 ist schließlich ein in die Werkzeugaufnahme 13 eingesetztes Werkzeug 14 dargestellt, das aus einem Bohrgerät 15 mit einer Bohrerspitze 16 besteht. Die Bohrerspitze befindet sich auf der genannten Werkzeugachse W. Um nun den Bearbeitungsvorgang durchzuführen, ist die Werkzeugaufnahme 13 verschieblich an dem Exzenterarm 12 festgelegt, wobei die Verschiebebewegung entlang einer zweiten Translationsachse Z erfolgt, die entlang der Werkzeugachse W verläuft.

Während alle dargestellten Achsen A, B, C, X und Y über (nicht dargestellte) Arretiermittel festlegbar sind, also die entlang dieser bzw. um diese Achsen verstellbaren Teile zueinander in vorgegebener Position festgelegt werden können, bleibt die Werkzeugaufnahme 13 entlang der zweiten Translationsachse W bzw. der Bohrachse Z bewegbar, um den Bearbeitungsvorgang durchführen zu können.

Die genannten Achsen sind jeweils über (nicht dargestellte) Aufnehmer hinsichtlich ihrer Orientierung bzw. Bewegung abtastbar, um digitalisierte Daten zu erzeugen, die es bei Anschließen an einen Rechner über eine beispielsweise serielle Schnittstelle ermöglichen, diese Ist-Daten mit vorgegebenen Sollwertdaten zu vergleichen, die von einer externen Diagnosesoftware erarbeitet und übertragen werden können. Die Istwerte können darüber hinaus auf eine (ebenfalls nicht dargestellte) Anzeigevorrichtung übertragen werden, wo sie den Sollwerten gegenübergestellt werden können, um das Einstellen der jeweiligen Achsen zu vereinfachen. Die erfassten Ist-Werte, die beim Bearbeiten des Werkstücks verwendet werden, können in einer (ebenfalls nicht dargestellten) Blackbox abgespeichert und manipulationsfrei archiviert werden. Durch die Digitalisierung der Achsen ist eine Genauigkeit von bis zu 0,01 mm möglich, wobei diese genauen Einstellungen zudem reproduzierbar sind.

ln der Zeichnung ist schließlich noch eine Halterung 17 für einen nicht dargestellten Fräsarm ersichtlich, wodurch man das auf der Modellaufnahmeplatte 5 angeordnete Werkstück zusätzlich mit einem Fräser bearbeiten kann.

Zusammenfassend bietet die Erfindung somit den Vorteil, die wesentlichen Verstellmöglichkeiten so aufeinander abstimmen zu können, dass die zugehörigen Achsen in dem Werkzeug zusammenfallen und damit das Verstellen einer Achse nicht automatisch dazu führt, dass sich der Bearbeitungsbereich durch einen zu großen Verschwenkbereich wie bei den bekannten exzentrisch angeordneten Werkzeugachsen ändert.

## Patentansprüche

1. Arbeitstisch für Zahntechniker und Zahnärzte und insbesondere zur Erstellung einer Implantats-Bohrschablone mit einer ein Werkstück zumindest mittelbar tragenden unteren Grundplatte (2) und einer zur unteren Grundplatte beweglich angeordneten Werkzeugaufnahme (13) für ein Werkzeug (14), wie insbesondere einen Bohrer, mit einer Werkzeugachse (W), wobei die Werkzeugaufnahme relativ zur unteren Grundplatte um zumindest zwei Schwenkachsen (A, B) und entlang von zumindest zwei Translationsachsen (Y, Z) verstell- und bewegbar angeordnet ist, wobei die beiden Schwenkachsen in einer gemeinsamen horizontalen Ebene liegen und sich unter einem Winkel in der Größenordnung von 90° schneiden, und wobei die erste Translationsachse (Y) in Horizontalrichtung orientiert ist und wobei die zweite Translationsachse (Z) identisch mit der Werkzeugachse (W) ist,
**dadurch gekennzeichnet,**
**dass** sich die beiden Schwenkachsen (A, B) und die Werkzeugachse (W) in einem gemeinsamen Schnittpunkt (O) schneiden.

2. Arbeitstisch nach zumindest Anspruch 1,
**dadurch gekennzeichnet,**
**dass** die erste Translationsachse (Y) entlang der ersten Schwenkachse (A) und somit ebenfalls durch den genannten gemeinsamen Schnittpunkt (O) verläuft.

3. Arbeitstisch nach zumindest einem der vorstehenden Ansprüche,
**dadurch gekennzeichnet,**
**dass** zumindest die beiden Schwenkachsen (A, B) Arretiermittel aufweisen zur Festlegung der zueinander bewegbaren Teile des Arbeitstisches.

4. Arbeitstisch nach zumindest Anspruch 3,
**dadurch gekennzeichnet,**
**dass** die Arretiermittel aus konischen Flächenpressungsabschnitten bestehen.

5. Arbeitstisch nach zumindest einem der vorstehenden Ansprüche,
**dadurch gekennzeichnet,**
**dass** die Schwenk- und/oder Translationsachsen derart abtastbar sind, dass digitalisierte Daten über die Schwenk- und/oder Translationsposition erzeugbar sind.

6. Arbeitstisch nach zumindest Anspruch 5,
**dadurch gekennzeichnet,**
**dass** die digitalisierten Daten an eine Rechnereinheit übertragbar sind.

7. Arbeitstisch nach zumindest Anspruch 5,
**dadurch gekennzeichnet,**
**dass** die digitalisierten Daten in einer Blackbox ablegbar und archivierbar sind.

8. Arbeitstisch nach zumindest einem der vorstehenden Ansprüche,
**dadurch gekennzeichnet.**
**dass** die Werkzeugaufnahme (13) relativ zur Grundplatte (2) um zumindest zwei Schwenkachsen (A, B) und entlang von zumindest drei Translationsachsen (X, Y, Z) verstellbar angeordnet ist.

9. Arbeitstisch nach zumindest Anspruch 8,
**dadurch gekennzeichnet,**
**dass** die drei Translationsachsen (X, Y, Z) den kartesischen Koordinaten im Raum entsprechend senkrecht zueinander angeordnet sind.

10. Arbeitstisch nach zumindest einem der vorstehenden Ansprüche,
**dadurch gekennzeichnet,**
**dass** das Werkstück auf einer Modellaufnahmeplatte (5) angeordnet ist und dass die Modellaufnahmeplatte um eine senkrecht zur Grundplatte (2) sich erstreckende vertikale Schwenkachse (C) schwenkbar angeordnet ist.

11. Arbeitstisch nach zumindest Anspruch 10,
**dadurch gekennzeichnet,**
**dass** die Modellaufnahmeplatte (5) über zusätzliche Stellglieder (6) relativ zur Grundplatte (2) in ihrer Neigung veränderbar angeordnet sind.
